(19) Europäisches Patentamt
European Patent Office
Office européen des brevets

(11) EP 0 899 236 A1

(12) # DEMANDE DE BREVET EUROPEEN

(43) Date de publication:
**03.03.1999 Bulletin 1999/09**

(51) Int Cl.[6]: **C01G 23/053**, A61K 7/42, C09C 1/36

(21) Numéro de dépôt: **98401986.9**

(22) Date de dépôt: **04.08.1998**

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **26.08.1997 FR 9710659**

(71) Demandeur: **L'OREAL**
**75008 Paris (FR)**

(72) Inventeur: **Remy, Christophe**
**94400 Vitry/S/Seine (FR)**

(74) Mandataire: **Dodin, Catherine**
**L'Oreal-D.P.I.,**
**90, rue du Général Roguet**
**92583 Clichy Cédex (FR)**

(54) **Procédé de préparation d'oxyde de titane photochrome, composé obtenu et composition le comprenant**

(57) La présente invention concerne un nouveau procédé de préparation d'un oxyde de titane photochrome, par traitement thermique d'un mélange hydrolysé de chlorure de titane et d'un précurseur d'un métal.

L'invention concerne également les composés photochromes de type oxyde de titane obtenus par ledit procédé, ainsi que les compositions notamment cosmétiques comprenant lesdits composés.



Printed by Jouve, 75001 PARIS (FR)

## Description

La présente invention se rapporte à l'amélioration des propriétés photochromes d'un oxyde de titane, grâce à un nouveau procédé de synthèse; l'invention concerne également le nouveau composé obtenu ainsi que son application dans le domaine des compositions cosmétiques notamment.

Les compositions cosmétiques, notamment les compositions de maquillage telles que les poudres libres ou compactes, les fonds de teint, les fards à joues ou à paupières, les rouges à lèvres ou les vernis à ongles, sont constituées d'un véhicule approprié et de différents agents de coloration destinés à conférer une certaine couleur auxdites compositions avant et/ou après leur application sur la peau, les muqueuses, les semi-muqueuses et/ou les phanères telles que les ongles ou les cheveux.
Pour créer des couleurs, on utilise aujourd'hui une gamme d'agents de coloration assez limitée et notamment des laques, des pigments minéraux et/ou des pigments nacrés. Les laques permettent d'obtenir des couleurs vives, mais pour la plupart sont instables à la lumière, à la température ou au pH. Certaines présentent également l'inconvénient de tacher la peau de manière disgracieuse après application, par dégorgement du colorant. Les pigments minéraux, en particulier les oxydes minéraux sont au contraire très stables mais donnent des couleurs plutôt ternes et pâles. Pour obtenir des effets colorés, on peut encore employer des pigments nacrés de couleurs variées, mais jamais intenses, qui permettent d'obtenir des effets irisés mais le plus souvent assez faibles.

Il a alors été proposé d'utiliser des composés photochromes dans les compositions de maquillage ou capillaire, de manière à obtenir des changements agréables et variables dans le 'rendu couleur' des maquillages de la peau et/ou des cheveux.
Les composés photochromes sont des composés qui possèdent la propriété de changer de couleur lorsqu'ils sont irradiés par une source lumineuse, puis de reprendre leur couleur initiale, ou une couleur proche, lorsque l'on arrête l'irradiation. De tels composés trouvent notamment une application particulièrement intéressante dans les compositions cosmétiques, en particulier dans les compositions de maquillage telles que les fonds de teint et les fards à joues ou à paupières. En effet, on a constaté que le 'rendu maquillage' d'une peau maquillée est différent selon que l'on se trouve en éclairage naturel ou en éclairage artificiel. Ainsi, un maquillage réalisé en éclairage artificiel paraîtra plus clair en lumière naturelle. A l'inverse, un maquillage réalisé à l'extérieur paraîtra plus sombre dans un endroit éclairé artificiellement.

Les propriétés photochromes d'un composé peuvent être caractérisées à l'aide des coordonnées trichromatiques (L, a, et b) de la manière décrite avant les exemples. Ces coordonnées permettent notamment la détermination d'un paramètre $\Delta E$ qui servira dans la présente demande à caractériser le photochromisme des composés selon l'invention et hors invention.
D'une manière générale, plus le paramètre $\Delta E$ est élevé, plus le composé est photochrome.

Dans l'art antérieur, il a notamment été proposé d'utiliser en cosmétique des composés photochromes organiques, tels que les composés de la famille des spiropyrannes ou des naphtooxazines.
Ces composés photochromes sont particulièrement intéressants dans la mesure où ils permettent d'obtenir un changement rapide de coloration du support sur lequel ils sont appliqués, lorsque ledit support est exposé aux UV par exemple, avec un retour rapide à la couleur initiale lorsque l'exposition aux UV cesse. On peut ainsi citer le brevet FR1604929 qui décrit des compositions cosmétiques notamment capillaires sous forme d'aérosol, qui contiennent des composés phototropes tels que des nitrobenzylpyridines, des thiosemicarbazones ou des dérivés de spiropyrannes. Après sprayage de ces compositions sur les cheveux et exposition à la lumière du soleil, on obtient une coloration bleu-violet qui redevient jaune pâle dans l'obscurité.

Il a également été proposé, par exemple par le brevet EP359909, des compositions cosmétiques comprenant des composés photochromes minéraux particulier, choisis parmi les oxydes métalliques, leurs hydrates et leurs complexes. En particulier, ce document mentionne l'utilisation d'oxyde de titane traité de manière à le rendre photochrome, dans des compositions de maquillage telles que des poudres et des fonds de teint.

On connaît par ailleurs, par le document US5176905, un procédé permettant l'obtention d'un oxyde de titane photochrome par mélange d'hydroxyde de fer (FeOOH) et de dioxyde de titane, et calcination à 750-850°C.

On connaît encore, par le document EP624553, un procédé de préparation d'oxyde de titane ayant un photochromisme amélioré, consistant à dissoudre un organotitane et un composé organique comprenant au moins un métal, dans un solvant organique, puis à hydrolyser le mélange, récupérer l'hydrolysat et à le calciner à une température de 550-700°C. On obtient ainsi un oxyde de titane photochrome, présentant une différence de couleur qui peut être quantifiée à l'aide du paramètre $\Delta E$ dont la valeur est d'au moins 10. La valeur $\Delta E$ est la différence mesurée entre la chromaticité avant irradiation et la chromaticité après irradiation pendant 1 heure, sous UV à 2 $mW/cm^2$.

On connaît également, par le document JP05/017152, un procédé de préparation d'un oxyde de titane photochrome, consistant à mélanger des organotitanes avec au moins un métal choisi parmi le fer, le chrome, le cuivre, le nickel, le vanadium ou le manganèse, puis à fritter ledit mélange en présence de composés du sodium. On peut ainsi obtenir un paramètre $\Delta E$ amélioré par rapport à l'art antérieur, et notamment supérieur à 10. Le paramètre $\Delta E$

est calculé de la même manière que dans EP624553.

On connaît encore, par l'article de Fitzpatrick et al., publié dans le journal 'Clays and clay minerals' volume 26, no3, pp.189-201 (1978), un procédé de préparation de coprécipités d'oxyde de titane et de fer, par hydrolyse; la poudre obtenue peut notamment être calcinée mais seulement jusqu'à une température de 250°C. Or, il a été constaté que les composés ainsi obtenus ne possèdent pas les propriétés photochromes recherchées.

Il subsiste toujours le besoin de disposer de composés photochromes permettant l'obtention d'un photochromisme important, notamment permettant d'obtenir un changement appréciable de la couleur du maquillage, c'est-à-dire un $\Delta E$ relativement important, notamment supérieur ou égal à 12.

La présente invention a pour but de proposer un procédé particulier permettant la préparation de tels composés.

La présente invention a donc pour objet un procédé de préparation d'un oxyde de titane photochrome, dans lequel :

- on prépare un mélange comprenant un chlorure de titane et un précurseur d'un métal choisi parmi le fer, le chrome, le cuivre, le nickel, le manganèse, le cobalt et le molybdène,
- on hydrolyse le mélange puis
- on traite thermiquement le mélange obtenu, à une température d'au moins 300°C de manière à obtenir un composé ayant un paramètre $\Delta E$ supérieur ou égal à 12.

L'invention a également pour objets le composé photochrome de type oxyde de titane susceptible d'être obtenu par le procédé ci-dessus, ainsi qu'une composition, notamment cosmétique, comprenant ledit composé.

Le procédé selon l'invention consiste donc à traiter thermiquement un mélange comprenant un chlorure de titane et au moins un précurseur d'un métal.

Le chlorure de titane peut notamment être $TiCl_4$.

Par 'précurseur d'un métal' on entend tout composé comprenant ledit métal et susceptible de le libérer.

On peut notamment employer un hydrate, un oxyde, un sel tel qu'un sulfate ou un chlorure dudit métal. Le métal est de préférence choisi parmi le fer, le chrome, le cuivre, le nickel, le manganèse, le cobalt ou le molybdène, seul ou en mélange. On emploie de préférence un chlorure ou un sulfate de fer.

De préférence, on utilise un précurseur de métal sous forme liquide ou sous forme pulvérulente.

Généralement, on choisit les quantités desdits composés de manière à obtenir un rapport 'équivalent oxyde métallique' : 'équivalent oxyde de titane' compris entre 0,05:100 et 10:100, de préférence entre 0,1:100 et 2:100.

On hydrolyse ensuite le mélange obtenu par addition d'une base de Brönsted, de préférence une base forte de type ammoniaque ou plus simplement à l'aide d'eau ou d'une solution aqueuse légèrement basique.

Ce traitement permet d'hydrolyser le chlorure de titane et de le transformer en 'gel de titane' (dénomination donnée par analogie avec les gels de silicone). On peut notamment effectuer l'hydrolyse à température ambiante, comprise entre 20 et 30°C. De préférence, on effectue l'hydrolyse jusqu'à l'obtention d'un mélange ayant un pH de 2 à 10, notamment de 5 à 6.

Le mélange peut ensuite être prétraité thermiquement, par exemple à 70-110°C, de préférence à 80-100°C, pendant 2-10 heures, de préférence 4-7 heures. On obtient alors un oxyde de titane au moins partiellement amorphe et qui ne présente pas les propriétés photochromes adéquates.

L'oxyde de titane se présente généralement sous forme de poudre en suspension dans le milieu réactionnel; on peut filtrer, laver et sécher ladite poudre selon les moyens usuels.

Le composé résultant est alors traité thermiquement à une température d'au moins 300°C, de préférence à une température comprise entre 400-800°C et préférentiellement entre 500-700°C, pendant un temps suffisant pour obtenir une calcination du composé, c'est-à-dire un oxyde de titane au moins partiellement cristallin et qui va présenter les propriétés photochromes recherchées.

La température ne doit toutefois pas être telle qu'elle permet la transformation du titane sous forme anatase en titane sous forme rutile. Il est en effet important de garder la forme cristallographique anatase pour l'oxyde de titane, la forme 'Rutile' ne conduisant pas à un photochromisme adéquat.

Le temps de traitement thermique peut notamment être de 2 à 8 heures, de préférence 3 à 5 heures, et dépend notamment de la température de calcination. L'homme du métier sait ajuster le couple 'température/temps' de calcination en fonction du résultat recherché, en se basant sur ses connaissances générales.

On obtient ainsi des oxydes de titane ayant de bonnes propriétés photochromes. Notamment, le paramètre $\Delta E$ des composés selon l'invention est au moins supérieur ou égal à 12, en particulier supérieur ou égal à 15, 20, voire à 25.

Le composé photochrome traité selon le procédé de l'invention peut être incorporé dans une composition, notamment cosmétique, en une quantité déterminable aisément par l'homme du métier sur la base de ses connaissances générales, et qui peut notamment être comprise entre 0,01 et 30% en poids par rapport au poids total de la composition, de préférence en une quantité de 1 à 15% en poids.

Ladite composition cosmétique peut se présenter sous la forme d'un produit à appliquer sur les muqueuses, les semi-muqueuses et/ou les tissus kératiniques tels que la peau et les phanères (ongles, cils, sourcils, poils et cheveux). Elle contient donc un milieu cosmétiquement acceptable, c'est-à-dire un milieu compatible avec toutes les matières kératiniques telles que la peau, les ongles, les cheveux, les cils et sourcils, les muqueuses et les semi-muqueuses, et toute autre zone cutanée du corps et du visage. Ledit milieu peut comprendre ou se présenter sous la forme de, notamment, une suspension, une dispersion, une solution en milieu solvant ou hydroalcoolique, éventuellement épaissie voire gélifiée; une émulsion huile-dans-eau, eau-dans-huile, ou multiple; un gel ou une mousse; un gel émulsionné; une dispersion de vésicules notamment lipidiques; une lotion biphase ou multiphase; un spray; une poudre libre, compacte ou coulée; une pâte anhydre. L'homme du métier pourra choisir la forme galénique appropriée, ainsi que sa méthode de préparation, sur base de ses connaissances générales, en tenant compte d'une part de la nature des constituants utilisés, notamment de leur solubilité dans le support, et d'autre part de l'application envisagée pour la composition.

Lorsque la composition se présente sous forme aqueuse, notamment sous forme de dispersion, d'émulsion ou de solution aqueuse, elle peut comprendre une phase aqueuse, qui peut comprendre de l'eau, une eau florale telle que l'eau de bleuet et/ou une eau minérale telle que l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevar-les-Bains, l'eau de Digne, l'eau de Lucas, l'eau de Maizières, l'eau de Neyrac-les-Bains, l'eau de Lons-le-Saunier, les Eaux Bonnes, l'eau de Roche-fort, l'eau de Saint Christau, l'eau des Fumades et l'eau de Tercis-les-bains. Ladite phase aqueuse peut comprendre de 0 % à 14 % en poids, par rapport au poids total de la phase aqueuse, d'un monoalcool inférieur en $C_2$-$C_6$ et/ou d'un polyol tel que le glycérol, le butylèneglycol, l'isoprène glycol, le propylèneglycol, le polyéthylèneglycol.

Lorsque la composition selon l'invention se présente sous la forme d'une émulsion, elle peut éventuellement comprendre en outre un tensioactif, de préférence en une quantité de 0,01 à 30% en poids par rapport au poids total de la composition.

Parmi les tensioactifs anioniques qui peuvent être utilisés seuls ou en mélange, on peut citer en particulier les sels alcalins, les sels d'ammonium, les sels d'amines ou les sels d'aminoalcools des composés suivants : les alcoylsulfates, alcoyléther sulfates, alcoylamides sulfates et éthers sulfates, alcoylarylpolyéthersulfates, monoglycérides sulfates, alcoylsulfonates, alcoylamides sulfonates, alcoylarylsulfonates, α-oléfines sulfonates, paraffines sulfonates, alcoylsulfosuccinates, alcoyléthersulfosuccinates, alcoylamides sulfosuccinates, alcoylsulfosuccinamates, alcoylsulfoacétates, alcoylpolyglycérol carboxylates, alcoylphosphates/alcoylétherphosphates, acylsarcosinates, alcoylpolypeptidates, alcoylamidopolypeptidates, acyliséthionates, alcoyllaurates. Le radical alcoyle ou acyle dans tous ces composés désigne généralement une chaîne de 12 à 18 atomes de carbone. On peut aussi citer les savons et les sels d'acides gras tels que les acides oléique, ricinoléique, palmitique, stéarique, les acides d'huile de coprah ou d'huile de coprah hydrogénée et notamment les sels d'amines tels que les stéarates d'amines; les acyl lactylates dont le radical acyle comprend 8-20 atomes de carbone; les acides carboxyliques d'éthers polyglycoliques répondant à la formule : Alk-$(OCH_2\text{-}CH_2)_n$-$OCH_2$-COOH sous forme acide ou salifiée où le substituant Alk correspond à une chaîne linéaire ayant de 12 à 18 atomes de carbone et où n est un nombre entier compris entre 5 et 15.

Parmi les tensioactifs non ioniques qui peuvent être utilisés seuls ou en mélange, on peut citer en particulier : les alcools, les alcoylphénols et acides gras polyéthoxylés, polypropoxylés ou polyglycérolés à chaîne grasse comportant 8 à 18 atomes de carbone; des copolymères d'oxydes d'éthylène et de propylène, des condensats d'oxyde d'éthylène et de propylène sur des alcools gras, des amides gras polyéthoxylés, des amines grasses polyéthoxylées, des éthanolamides, des esters d'acides gras de glycol, des esters d'acides gras du sorbitan oxyéthylénés ou non, des esters d'acides gras du saccharose, des esters d'acides gras de polyéthylèneglycol, des triesters phosphoriques, des esters d'acides gras de dérivés de glucose; les alkylpolyglycosides et les alkylamides des sucres aminés; les produits de condensation d'un α-diol, d'un monoalcool, d'un alcoylphénol, d'un amide ou d'un diglycolamide avec le glycidol ou un précurseur de glycidol.

La composition selon l'invention peut également comprendre 0 à 5 % en poids, par rapport au poids total de l'émulsion, d'au moins un co-émulsionnant qui peut être choisi parmi le monostéarate de sorbitan oxyéthyléné, des alcools gras tels que l'alcool stéarylique ou l'alcool cétylique, ou des esters d'acides gras et de polyols tels que le stéarate de glycéryle.

La composition selon l'invention peut encore comprendre un ou plusieurs agents épaississants dans des concentrations préférentielles allant de 0 à 6 % en poids, par rapport au poids total de la composition, choisis parmi:

- les biopolymères polysaccharidiques comme la gomme de xanthane, la gomme de caroube, la gomme de guar, les alginates, les celluloses modifiées telles que l'hydroxyéthylcellulose, la méthylcellulose, l'hydroxypropylcellulose et la carboxyméthylcellulose, les dérivés de l'amidon, les dérivés d'éthers de cellulose possédant des groupes ammonium quatemaires, les polysaccharides cationiques;
- les polymères synthétiques comme les acides polyacryliques tels que les polymères poly(méth)acrylates de glycéryl tels que l'HISPAGEL ou le LUBRAGEL des sociétés HISPANO QUIMICA ou GARDIAN, la polyvinylpyrrolidone, l'alcool polyvinylique, les polymères réticulés d'acrylamide et d'acrylate d'ammonium tels que le PAS 5161 ou BOZEPOL C de HOECHST; les copolymères acrylate/octylacrylamide tels que le Dermacryl de National Starch; les polymères à base de polyacrylamide tels que le SEPIGEL 305 de SEPPIC, les polymères réticulés d'acrylamide et de chlorure de méthacryloyloxyéthyltriméthylammonium tels que le SALCARE SC 92 de ALLIED COLLOIDS,
- le silicate de magnésium et d'aluminium.

Selon l'application envisagée, la composition peut comprendre en outre un polymère filmogène. Ceci est notamment le cas lorsque l'on souhaite préparer une composition de type vernis à ongles, mascara, eye-liner ou composition capillaire de type laque. Les polymères peuvent être dissous ou dispersés dans le milieu cosmétiquement acceptable. En particulier, le polymère peut être présent sous forme de solution dans un solvant organique ou sous forme de dispersion aqueuse de particules de polymère filmogène. Ledit polymère peut être choisi parmi la nitrocellulose, l'acétobutyrate de cellulose, les butyralpolyvinyliques, les résines alkydes, les polyesters, les acryliques, les vinyliques, et/ou les polyuréthannes. On peut en particulier citer les copolymères d'acide (méth)acrylique et d'au moins un monomère ester d'acide (méth)acrylique linéaire, ramifié ou cyclique et/ou d'au moins un monomère amide d'acide (méth)acrylique mono ou di-substitué linéaire, ramifié ou cyclique; les copolymères acide (méth)acrylique/(meth)acrylate de tertio-butyle et/ou (méth)acrylate d'isobutyle/(méth)acrylate d'alkyle en $C_1$-$C_4$; les terpolymères et tétrapolymères acide (meth)acrylique/acrylate d'éthyle/ méthacrylate de méthyle; les tétrapolymères méthacrylate de méthyle/acrylate de butyle ou d'éthyle/acrylate ou méthacrylate d'hydroxyéthyle ou de 2-hydroxypropyle/acide (meth)acrylique; les copolymères d'acide acrylique et de méthacrylate d'alkyle en $C_1$-$C_4$ ; les terpolymères de vinyl pyrrolidone, d'acide acrylique et de méthacrylate d'alkyle en $C_{1-20}$ ; les copolymères amphotères; les esters vinyliques d'acide ramifiés; les esters vinyliques d'acide benzoïque; les copolymères d'acide (meth)acrylique et d'au moins un monomère oléfinique; les copolymères de monoacide vinylique et/ou de monoacide allylique. Parmi les résines, on peut citer les résines du type aryl-sulfonamide formaldéhyde ou aryl-sulfonamide époxy; les résines du type acrylique, styrénique, acrylate-styrénique et vinylique.

La composition peut également comprendre au moins un plastifiant, tel que le phosphate de tricrésyle, le benzoate de benzyle, le citrate de triéthyle, le citrate de tributyle, l'acétylcitrate de triéthyle, l'acétylcitrate de triétyl-2 hexyle, le camphre; les éthers de glycol; l'huile de ricin oxyéthylénée à 40 moles d'oxyde d'éthylène; le propylène glycol; le butyl glycol ; l'éthylène glycol monométhyléther acétate; les éthers de propylène glycol; les éthers esters de propylène glycol et d'éthylène glycol; les esters de diacides tels que les phtalates et adipates de diéthyle, dibutyle et de diisopropyle, les tartrates de diéthyle et dibutyle, les succinates de diéthyle et de dibutyle, les sébacates de diéthyle et de dibutyle, les phosphates de diéthyle, de dibutyle et de diéthyl-2 hexyle, l'acétyl citrate de diéthyle ou de dibutyle; les esters de glycérol. Les plastifiants peuvent être généralement présents à une teneur allant de 1 % à 40 % en poids par rapport au poids total de la composition.

La composition selon l'invention peut également comprendre une phase grasse, notamment constituée de corps gras liquides à 25°C, tels que des huiles d'origine animale, végétale, minérale ou synthétique; de corps gras solides à 25°C tels que des cires d'origine animale, végétale, minérale ou synthétique; de corps gras pâteux; de gommes; de leurs mélanges.

Les compositions selon l'invention peuvent ainsi comprendre des huiles volatiles, qui s'évaporeront au contact de la peau, mais dont la présence, dans la composition cosmétique, est utile car elles facilitent l'étalement de la composition lors de l'application sur la peau. De tels agents d'étalement appelés ici "huiles volatiles", sont généralement des huiles ayant, à 25°C, une tension de vapeur saturante au moins égale à 0,5 millibar (soit 50 Pa). De préférence, on utilise des huiles dont le point éclair est suffisamment élevé pour permettre l'utilisation de ces huiles en formulation, et suffisamment bas pour obtenir l'effet évanescent souhaité. On emploie de préférence des huiles dont le point éclair est de l'ordre de 40-100°C. On peut ainsi citer les huiles siliconées volatiles, telles que :

- les silicones volatiles cycliques ayant de 3 à 8 atomes de silicium et de préférence de 4 à 6. Il s'agit par exemple de la cyclotétradiméthylsiloxane, de la cyclopentadiméthylsiloxane ou de la cyclohexadiméthylsiloxane,
- les cyclocopolymères du type diméthylsiloxane/méthylalkylsiloxane, tels que la SILICONE FZ 3109 vendue par la société UNION CARBIDE , qui est un cyclocopolymère diméthylsiloxane/méthyloctylsiloxane,
- les silicones volatiles linéaires ayant de 2 à 9 atomes de silicium. Il s'agit par exemple de l'hexaméthyldisiloxane, de l'hexylheptaméthyltrisiloxane ou de l'octylheptaméthyltrisiloxane.

On peut également citer les huiles volatiles hydrocarbonées, telles que les isoparaffines et notamment l'isododécane; et des huiles fluorées comme celle commercialisée sous la dénomination GALDEN® (MONTEFLUOS).

On peut également utiliser des huiles non volatiles, parmi lesquelles on peut citer:

- les polyalkyl($C_1$-$C_{20}$) siloxanes et notamment ceux à groupements terminaux triméthylsilyle, de préférence ceux

dont la viscosité est inférieure à 0,06 $m^2/s$ parmi lesquels on peut citer les polydiméthylsiloxanes linéaires et les alkylméthylpolysiloxanes tels que la cétyldiméthicone (nom CTFA),

- les silicones modifiées par des groupements aliphatiques et/ou aromatiques, éventuellement fluorés, ou par des groupements fonctionnels tels que des groupements hydroxyles, thiols et/ou amines.
- les huiles de silicone phénylées, notamment celles de formule :

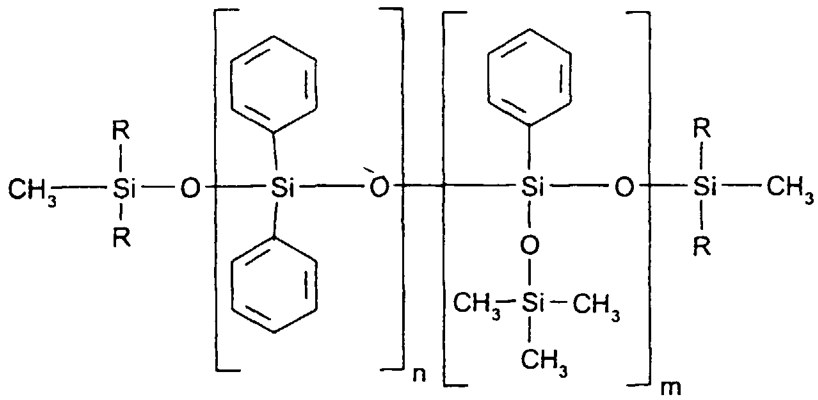

dans laquelle R est un radical alkyle en C1-C30, un radical aryle ou un radical aralkyle, n est un nombre entier compris entre 0 et 100, et m est un nombre entier compris entre 0 et 100, sous réserve que la somme est comprise entre 1 et 100.

- les huiles d'origine animale, végétale ou minérale, et notamment les huiles animales ou végétales formées par des esters d'acide gras et de polyols, en particulier les triglycérides liquides, par exemple les huiles de tournesol, de maïs, de soja, de courge, de pépins de raisin, de sésame, de noisette, d'abricot, d'amandes ou d'avocat; les huiles de poisson, le tricaprocaprylate de glycérol, ou les huiles végétales ou animales de formule $R_1COOR_2$ dans laquelle $R_1$ représente le reste d'un acide gras supérieur comportant de 7 à 19 atomes de carbone et $R_2$ représente une chaîne hydrocarbonée ramifiée contenant de 3 à 20 atomes de carbone, par exemple, l'huile de Purcellin; l'huile de paraffine, de vaseline, le perhydrosqualène, l'huile de germes de blé, de calophyllum, de sésame, de macadamia, de pépins de raisin, de colza, de coprah, d'arachide, de palme, de ricin, de jojoba, d'olive ou de germes de céréales; des esters d'acides gras; des alcools; des acétylglycérides; des octanoates, décanoates ou ricinoléates d'alcools ou de polyalcools; des triglycérides d'acides gras; des glycérides;
- les huiles fluorées et perfluorées.

La composition selon l'invention peut comprendre en outre d'autres corps gras, qui peuvent être choisis par l'homme du métier sur base de ses connaissances générales, de manière à conférer à la composition finale les propriétés souhaitées, par exemple en consistance et/ou en texture. Ces corps gras additionnels peuvent être des cires, des gommes et/ou des corps gras pâteux d'origine animale, végétale, minérale ou synthétique, ainsi que leurs mélanges.
On peut notamment citer :

- les gommes de silicones,
- les cires d'origine animale, végétale, minérale ou synthétique telles que les cires microcristallines, la paraffine, le pétrolatum, la vaseline, l'ozokérite, la cire de montan; la cire d'abeilles, la lanoline et ses dérivés; les cires de Candellila, d'Ouricury, de Carnauba, du Japon, le beurre de cacao, les cires de fibres de lièges ou de canne à sucre; les huiles hydrogénées concrètes à 25°C, les ozokérites, les esters gras et les glycérides concrets à 25°C; les cires de polyéthylène et les cires obtenues par synthèse de Fischer-Tropsch; des huiles hydrogénées concrètes à 25°C; des lanolines; des esters gras concrets à 25°C; les cires de silicone; les cires fluorées.

La composition selon l'invention peut également comprendre un ou plusieurs solvants organiques cosmétiquement acceptables (tolérance, toxicologie et toucher acceptables). Ces solvants organiques peuvent représenter de 0 % à 98 % du poids total de la composition et peuvent être choisis dans le groupe constitué par les solvants organiques hydrophiles, les solvants organiques lipophiles, les solvants amphiphiles ou leurs mélanges.
Parmi les solvants organiques hydrophiles, on peut citer par exemple des monoalcools inférieurs linéaires ou ramifiés ayant de 1 à 8 atomes de carbone comme l'éthanol, le propanol, le butanol, l'isopropanol, l'isobutanol ; des polyéthylène

glycols ayant de 6 à 80 oxydes d'éthylène ; des polyols tels que le propylène glycol, l'isoprène glycol, le butylène glycol, le glycérol, le sorbitol ; les mono- ou di-alkyle d'isosorbide dont les groupements alkyle ont de 1 à 5 atomes de carbone ; les éthers de glycol comme le diéthylène glycol mono-méthyl ou mono-éthyl éther et les éthers de propylène glycol comme le dipropylène glycol méthyl éther. Comme solvants organiques amphiphiles, on peut citer des polyols tels des dérivés de polypropylène glycol (PPG) tels que les esters de polypropylène glycol et d'acide gras, de PPG et d'alcool gras comme le PPG-23 oléyl éther et le PPG-36 oléate. Comme solvants organiques lipophiles, on peut citer par exemple les esters gras tels que l'adipate de diisopropyle, l'adipate de dioctyle, les benzoates d'alkyle, le myristate d'isopropyle, le palmitate d'isopropyle, le stéarate de butyle, le laurate d'hexyle, l'isononanoate d'isononyle, le palmitate de 2-éthyl hexyle, le laurate de 2-hexyl-décyle, le palmitate de 2-octyl-décyle, le myristate de 2-octyl-dodécyle, le succinate de di-(éthyl-2-hexyle), le malate de diisostéaryle, le lactate de 2-octyl-dodécyle, le triisostéarate de glycérine, le triisostéarate de diglycérine.

La composition peut comprendre en outre une phase particulaire, qui peut comprendre des pigments et/ou des nacres et/ou des charges habituellement utilisés dans les compositions cosmétiques. Par pigments, il faut comprendre des particules blanches ou colorées, minérales ou organiques, destinées à colorer et/ou opacifier la composition. Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage. Par nacres, il faut comprendre des particules irisées qui réfléchissent la lumière.

Les pigments peuvent être présents dans la composition à raison de 0 à 15% en poids de la composition finale, et de préférence à raison de 8 à 10% en poids. Ils peuvent être blancs ou colorés, minéraux et/ou organiques, de taille usuelle ou nanométrique. On peut citer les dioxydes de titane, de zirconium ou de cérium, ainsi que les oxydes de zinc, de fer ou de chrome, le bleu ferrique, l'hydrate de chrome, le noir de carbone, les outremers (polysulfures d'aluminosilicates), le pyrophosphate de manganèse et certaines poudres métalliques telles que celles d'argent ou d'aluminium, le noir de carbone. On peut encore citer les laques couramment employées pour conférer aux lèvres et à la peau un effet de maquillage, qui sont des sels de calcium, de baryum, d'aluminium ou de zirconium, de colorants acides.

Les nacres peuvent être présentes dans la composition à raison de 0 à 20% en poids, de préférence à un taux de l'ordre de 8 à 15% en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.

Les charges, qui peuvent être présentes à raison de 0 à 30% en poids, de préférence 5 à 15%, dans la composition, peuvent être minérales ou de synthèse, lamellaires ou non lamellaires. On peut citer le talc, le mica, la silice, le kaolin, les poudres de Nylon et de polyéthylène, le Téflon, l'amidon, le nitrure de bore, les microsphères de polymère telles que l'Expancel (Nobel Industrie), le polytrap (Dow Corning) et les microbilles de résine de silicone (Tospearls de Toshiba, par exemple), le carbonate de calcium précipité, le carbonate ou l'hydrocarbonate de magnésium, des savons métalliques dérivés d'acides organiques carboxyliques ayant de 8 à 22 atomes de carbone.

Selon le type de formulation, la phase pulvérulente peut représenter de 0,01 à 99% en poids de la composition.

La composition peut en outre comprendre un colorant, notamment un colorant organique naturel tel que le carmin de cochenille, et/ou un colorant de synthèse tel que les colorants halogéno-acides, azoïques, anthraquinoniques. On peut également citer des colorants minéraux tels que le sulfate de cuivre.

La composition peut comprendre en outre tout additif usuellement utilisé dans le domaine cosmétique, tel que des antioxydants, des parfums, des huiles essentielles, des conservateurs, des actifs cosmétiques lipophiles ou hydrophiles, des hydratants, des vitamines, des acides gras essentiels, des sphingolipides, des agents autobronzants tels que la DHA, des filtres solaires, des agents antimousses, des agents séquestrants, des antioxydants.

Bien entendu l'homme du métier veillera à choisir les éventuels composés complémentaires, et/ou leur quantité, de manière telle que les propriétés avantageuses de la composition selon l'invention ne soient pas, ou substantiellement pas, altérées par l'adjonction envisagée.

Les compositions cosmétiques peuvent se présenter sous la forme d'un produit de soin et/ou de maquillage de la peau, un produit solaire ou autobronzant, voire un produit capillaire. En particulier, elles trouvent une application particulière dans le domaine des rouges à lèvres, des fonds de teint, des fards à joues ou à paupières, des poudres libres ou compactes, des crèmes teintées, des eye-liners, des mascaras et des vernis à ongles aqueux ou solvant.

L'invention est illustrée plus en détail dans les exemples suivants.

<u>Méthode de détermination du paramètre ΔE à l'aide des coordonnées trichromatiques</u>

Le composé en poudre est tassé dans une coupelle métallique.

On mesure les coordonnées trichromatiques (L0, a0, b0) de la poudre tassée à l'aide d'un colorimètre Minolta CR300.

On irradie la coupelle pendant 30 minutes à l'aide d'une lampe UV à 365 nm, puis l'on mesure les nouvelles coordonnées (L30, a30 et b30) qui reflètent le changement de couleur suite à l'irradiation.

On définit le paramètre ΔE de la manière suivante; ce paramètre est caractéristique de l'activité photochrome du composé; plus ce paramètre sera important, plus le matériau sera photochrome.

$$\Delta E = [(L30 - L0)^2 + (a30 - a0)^2 + (b30 - b0)^2]^{1/2}$$

Exemple 1

On prépare un mélange comprenant 15 ml de chlorure de titane ($TiCl_4$) et 0,22 g de chlorure de fer ($FeCl_3$) en solution aqueuse à 41% en poids.
On ajoute goutte-à-goutte de l'ammoniaque à 10%, sous agitation, jusqu'à l'obtention d'un pH de 5,5.
On chauffe le mélange sous reflux à 90°C pendant 5 heures, puis on continue l'agitation à température ambiante pendant 24 heures. On filtre le mélange, lave le filtrat et sèche à 55°C pendant 24 heures.
On obtient une poudre que l'on calcine pendant 4 heures à 600°C.
On obtient de l'oxyde de titane dopé au fer.

Exemple 2

Différents procédés de synthèse d'oxyde de titane photochrome dopé au fer ont été réalisés afin de comparer les propriétés photochromes des composés obtenus.
Pour chaque mode de synthèse, une étude a été réalisée de façon à trouver les paramètres expérimentaux fournissant les meilleures propriétés.
Le tableau ci-après indique la valeur maximale de ΔE obtenue pour chaque mode de synthèse, et qui traduit, à priori, les propriétés photochromes optimales susceptibles d'être obtenues avec chaque mode de synthèse.

| Mode de synthèse | ΔE |
|---|---|
| A | 10 |
| B | 9 |
| C | 9 |
| D | 15 |
| E | 12 |
| F (invention) | 22 |

A : calcination à 900°C pendant 4 heures d'oxyde de titane et de chlorure de fer ($FeCl_3$)
B : dispersion aqueuse d'oxyde de titane et de chlorure de fer, neutralisation avec NaOH, filtration et calcination à 800°C pendant 4 heures.
C : dispersion aqueuse d'oxyde de titane et de chlorure de fer, neutralisation avec NaOH, ajout d'urée, filtration et calcination à 800°C pendant 4 heures.
D : synthèse à partir d'oxysulfate de titane (voir EP526712)
E : synthèse à partir d'alcoxyde de titane (voir EP624553)
F : exemple 1

On constate donc que le procédé selon l'invention permet bien d'obtenir un oxyde de titane photochrome ayant des propriétés améliorées par rapport à l'art antérieur.

Exemple 3

On recommence le procédé de préparation selon l'exemple 1 en faisant varier la température de calcination, les autres paramètres restant inchangés.
On obtient les résultats suivants :

| Température | ΔE |
|---|---|
| 300°C | 6 |
| 400°C | 23 |
| 600°C | 29 |
| 800°C | 2 |

On constate donc que le choix de la température de calcination est important pour obtenir de bonnes propriétés photochromes.

Exemple 4

On recommence le procédé de préparation selon l'exemple 1 en faisant varier la quantité de fer présent dans l'oxyde de titane, les autres paramètres restant inchangés.
On obtient les résultats suivants :

| % de fer (en poids) | ΔE |
|---|---|
| 0,1% | 6 |
| 0,4% | 29 |
| 0,8% | 15 |
| 1,0% | 14 |

Exemple 5

On prépare une poudre compactée ayant la composition suivante :

- talc 30 g
- mica 20 g
- poudre de Nylon 16 g
- stéarate de zinc 5 g
- oxyde de fer 2 g
- oxyde de titane selon l'exemple 1 10 g
- liant gras qsp 100 g

On obtient une composition ayant de bonnes propriétés cosmétiques.

**Revendications**

1. Procédé de préparation d'un oxyde de titane photochrome, dans lequel :

   - on prépare un mélange comprenant un chlorure de titane et un précurseur d'un métal choisi parmi le fer, le chrome, le cuivre, le nickel, le manganèse, le cobalt et le molybdène,
   - on hydrolyse ledit mélange et
   - on traite thermiquement le mélange obtenu, à une température d'au moins 300°C de manière à obtenir un composé ayant un paramètre ΔE supérieur ou égal à 12.

2. Procédé selon la revendication 1 dans lequel le composé final présente un ΔE supérieur ou égal à 15, de préférence supérieur ou égal à 20, préférentiellement supérieur ou égal à 25.

3. Procédé selon l'une des revendications précédentes, dans lequel le chlorure de titane employé est $TiCl_4$.

4. Procédé selon l'une des revendications précédentes, dans lequel le précurseur de métal est choisi parmi les hydrates, oxydes, sels tels que sulfate ou chlorure, dudit métal.

5. Procédé selon l'une des revendications précédentes, dans lequel le précurseur de métal est un chlorure de fer ou un sulfate de fer.

6. Procédé selon l'une des revendications précédentes, dans lequel les quantités de chlorure de titane et de précurseur de métal sont choisies de manière à obtenir un rapport'équivalent oxyde métallique' : 'équivalent oxyde de titane' compris entre 0,05:100 et 10:100, de préférence entre 0,1:100 et 2:100.

**7.** Procédé selon l'une des revendications précédentes, dans lequel l'hydrolyse est effectuée par addition d'une base de Brönsted, de préférence une base forte de type ammoniaque, ou à l'aide d'eau ou d'une solution aqueuse légèrement basique.

**8.** Procédé selon l'une des revendications précédentes, dans lequel le traitement thermique est effectué à une température comprise entre 400-800°C, préférentiellement entre 500-700°C.

**9.** Procédé selon l'une des revendications précédentes, dans lequel le temps de traitement thermique est de 2 à 8 heures, de préférence 3 à 5 heures.

**10.** Composé photochrome de type oxyde de titane susceptible d'être obtenu par le procédé selon l'une des revendications 1 à 9.

**11.** Composition notamment cosmétique comprenant le composé selon la revendication 10.

**12.** Composition selon la revendication 11 dans laquelle le composé photochrome est présent en une quantité comprise entre 0,01 et 30% en poids par rapport au poids total de la composition, de préférence en une quantité de 1 à 15% en poids.

**13.** Composition selon l'une des revendications 11 à 12, se présentant sous la forme d'un produit de soin et/ou de maquillage de la peau, un produit solaire ou autobronzant, un produit capillaire.

**14.** Composition selon l'une des revendications 11 à 13, se présentant sous la forme d'un rouge à lèvres, d'un fond de teint, d'un fard à joues ou à paupières, d'une poudre libre ou compacte, d'une crème teintée, d'un eye-liner, d'un mascara ou d'un vernis à ongles.

Office européen des brevets

# RAPPORT DE RECHERCHE EUROPEENNE

Numéro de la demande
EP 98 40 1986

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A | EP 0 359 909 A (SHISEIDO CO LTD) 28 mars 1990<br>* page 3 - page 5 *<br>* page 14 *<br>--- | 1,3-6,8, 10-14 | C01G23/053<br>A61K7/42<br>C09C1/36 |
| A | EP 0 526 712 A (SHISEIDO CO LTD) 10 février 1993<br>* page 6 - page 7; revendications 2-9 *<br>--- | 1-14 | |
| A | EP 0 624 553 A (SUMITOMO CHEMICAL CO) 17 novembre 1994<br>* le document en entier *<br>--- | 1-14 | |
| A | DE 43 02 896 A (DEGUSSA) 4 août 1994<br>* le document en entier *<br>--- | 1,3-6 | |
| A | DATABASE WPI<br>Section Ch, Week 9715<br>Derwent Publications Ltd., London, GB;<br>Class D21, AN 97-161384<br>XP002065946<br>& JP 09 030933 A (SHOKUBAI KASEI KOGYO KK)<br>, 4 février 1997<br>* abrégé *<br>----- | 1,3-7,11 | DOMAINES TECHNIQUES RECHERCHES (Int.Cl.6)<br>C01G<br>A61K<br>C09C |

Le présent rapport a été établi pour toutes les revendications

| Lieu de la recherche | Date d'achèvement de la recherche | Examinateur |
|---|---|---|
| LA HAYE | 1 décembre 1998 | LIBBERECHT, E |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)